# FASCICULE DE BREVET EUROPEEN

(11) **EP 1 698 686 B1**
(45) Date de publication et mention de la délivrance du brevet: **07.11.2012**
(21) Numéro de dépôt: 05292426.3
(22) Date de dépôt: 14.11.2005
(51) Int. Cl.: C11C 3/00, C07C 11/02, C07C 11/12, C07C 67/333, C07C 2/10, C07C 6/04, C10G 50/00, C10G 69/12

(54) **Procédé de co-production d'oléfines et d'esters par éthénolyse de corps gras insatures dans des liquides ioniques non-aqueux**
Gleichzeitige Herstellung von Olefinen und Estern durch Ethenolyse von ungesättigten Fetten in nicht-wässrigen ionischen Flüssigkeiten.
Co-Production of olefins and esters via ethenolysis of unsaturated fats in non-aqueous ionic liquids.

(30) Priorité: 23.11.2004 FR 0412414
(43) Date de publication de la demande: 06.09.2006
(73) Titulaire: Institut Français du Pétrole, 92852 Rueil Malmaison Cedex (FR)
(72) Inventeur: Thuriez, Cyril, 45100 Orleans (FR); Olivier-Bourbigou, Hélène, 69230 Saint Genis Laval (FR); Dixneuf, Pierre, 35000 Rennes (FR); Hillion, Gérard, 95220 Herblay (FR)

(56) Documents cités:
- US-A- 4 545 941
- US-A- 5 475 159
- US-A- 5 675 051
- US-B1- 6 756 500
- WARWEL S ET AL: "Polymers and surfactants on the basis of renewable resources" CHEMOSPHERE, PERGAMON PRESS, OXFORD, GB, vol. 43, 2001, pages 39-48, XP002262684 ISSN: 0045-6535
- DATABASE CAPLUS CHEMICAL ABSTRACTS SERVICE, COLUMBUS, OHIO, US; XP002340304 Database accession no. 1984:432612 & V.O.POLOTNYUK ET AL.: "use of a photoionization detector for identification of components of complex hydrocarbon mixtures" ZHURNAL ANALITICHESKOI KHIMII, vol. 39, no. 3, 1984, pages 529-532,
- DATABASE CAPLUS CHEMICAL ABSTRACTS SERVICE, COLUMBUS, OHIO, US; XP002340305 Database accession no. 1996:437700 & A. HEFETZ ET AL.: "The exocrinology of the queen bumble bee Bombus terrestris" ZEITSCHRIFT FÜR NATURFORSCHUNG, vol. 51, no. 5/6, 1996, pages 409-422,
- H. OLIVIER-BOURGIGOU ET AL.: "Ionic-liquids: perspectives for organic and catalytic reactions." JOURNAL OF MOLECULAR CATALYSIS A, vol. 182-183, 2002, pages 419-437, XP002340268

## Description

L'invention concerne la coproduction d'oléfines et d'esters par métathèse par l'éthylène (ou éthénolyse) de corps gras insaturés, en présence d'un catalyseur et d'au moins un liquide ionique non-aqueux.

### Art antérieur

La réaction de métathèse des oléfines est une réaction bien connue de la chimie organique. Cette réaction, qui s'effectue en présence d'un système catalytique approprié, consiste en l'échange de groupes alkylidènes entre deux oléfines selon les équations suivantes :
1. Premier cas, dit de "self métathèse" ou "homométathèse" (c'est-à-dire de métathèse d'une molécule d'oléfine sur une molécule de la même oléfine) : 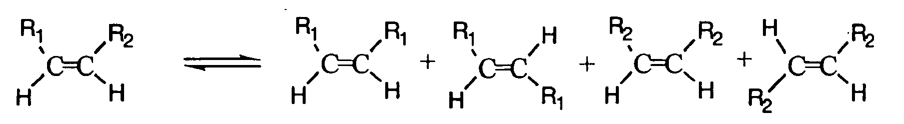
2. Second cas, dit de "métathèse croisée" ou "cross-metathesis" (c'est-à-dire de métathèse entre deux oléfines différentes) :

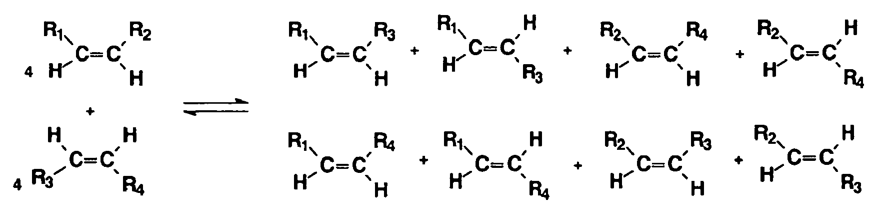

La réaction de métathèse des oléfines est une réaction équilibrée. Elle peut se produire en présence d'une grande variété de catalyseurs, le plus souvent à base de métaux de transition des groupes IVA à VIII, parmi lesquels le tungstène, le molybdène, le rhénium et le ruthénium, soit en phase homogène, soit en phase hétérogène. De nombreuses revues et ouvrages scientifiques traitent de cet aspect. On citera par exemple :
- K. J. Ivin and J. C. Mol dans "Olefin Metathesis and Metathesis Polymerization", San Diego, Academic Press (1997) ;
- "Handbook of Metathesis" R.H. Grubbs (Ed) Wiley-VCH, Weinheim (2003) ;
- J .C. Mol "Industrial Applications of Olefin Metathesis" J. Mol. Catal. 213, 39 (2004) ;
- D. Séméril and P.H. Dixneuf, dans "Novel Metathesis Chemistry: Well-Defined Initiator Systems for Specialty Chemical Synthesis, Tailored Polymers and Advanced Material Applications" Y. Imamoglu and L. Bencze (Eds) Kluwer Academic Publishers, The Netherlands (2003) 1-21.

Si l'une des oléfines est un ester d'acide gras insaturé, représenté par exemple par l'oléate de méthyle, la réaction conduit à l'obtention d'une ou plusieurs oléfines et d'un ou plusieurs esters insaturés. La réaction peut s'écrire :

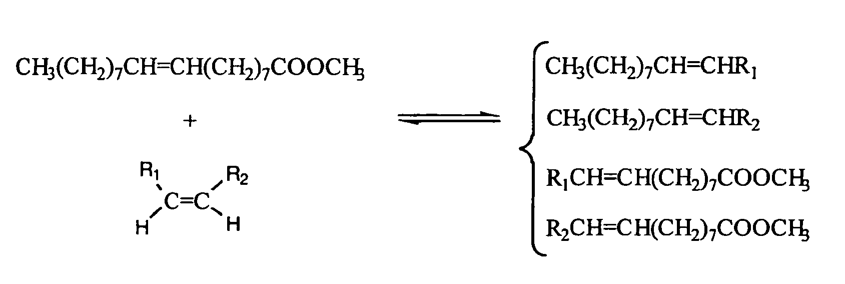

Si l'oléfine R₁HC=CHR₂ est l'éthylène et qu'elle est utilisée en un excès suffisant pour déplacer l'équilibre, la réaction peut conduire à une alpha-oléfine et à un ester insaturé, qui sont, dans le cas particulier envisagé, le décène-1 et le 9-décénoate de méthyle. La réaction particulière peut s'écrire :

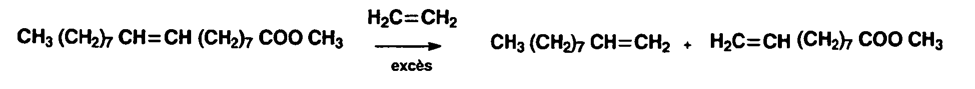

Cette réaction est potentiellement d'un grand intérêt, car elle permet d'envisager la fabrication à partir d'une matière première pour l'essentiel d'origine végétale ou animale, donc renouvelable, des produits tels qu'une alpha-oléfine, dans le cas particulier envisagé le décène-1, qui est un intermédiaire recherché pour la pétrochimie, habituellement entièrement fabriqué à partir de matières premières fossiles comme l'éthylène.

Cette réaction de métathèse par éthénolyse appliquée à des esters d'acides gras a été reportée dans de nombreuses publications ou revues scientifiques. À titre d'exemples, on peut citer, parmi les références les plus récentes :
- "Application of Olefin Metathesis in Oleochemistry: an Example of Green Chemistry" par J. C. Mol, Green Chemistry, 4, 5-13 (2002) ;
- "The Metathesis of Polyunsaturated Fatty Esters Using the Homogeneous W(O-2,6-C6H3X2)2Cl4/Me4Sn Catalytic Systems" par B.B. Marvey et coll. J. Mol. Catal. 213, 151-157 (2004) ;
- "Technological and Economical Aspects of the Metathesis of Unsaturated Esters" par M. Sibeijn et coll. JAOCS, 71, 6 (1994) ;
- "Polymer and Surfactants on the Basis of Renewable Resources" par S. Warwel et coll. Chemosphere 43, 39-48 (2001) ;
- "Catalysis Metathesis of Unsaturated Fatty Acid Esters and Oils" par J.C. Mol, Topics in Catalysis, 27, 1, (2004).

Différents types de catalyseurs ont été décrits pour réaliser une telle transformation. Les premiers systèmes sont homogènes, à base de tungstène et de tétraalkylétains, par exemple WCI₆/SnMe₄. Puis les systèmes hétérogènes à base de rhenium activé par des tétraaklylétains. Cependant, ces systèmes ont l'inconvénient de mettre en oeuvre des co-catalyseurs, en général à base d'étain qui peuvent contaminer les produits de la réaction. Plus récemment, les systèmes homogènes "bien définis", n'utilisant pas de co-catalyseur, et à base de métal-carbènes (M=C) ont été décrits, le métal étant le tungstène ou le molybdène. Cependant, la principale difficulté rencontrée avec l'ensemble de ces systèmes reste leur faible compatibilité avec les groupes fonctionnels tels que les acides ou les esters tels que ceux présents dans les huiles végétales. Cela entraîne en général une faible activité et une désactivation rapide de ces systèmes catalytiques.

Les complexes à base de ruthénium s'avèrent rapidement très intéressants en raison de leur tolérance vis-à-vis d'une large gamme de groupes fonctionnels. Cette propriété, couplée avec une activité souvent élevée, explique leur développement important dans le domaine de la synthèse des polymères et pour la synthèse organique.

Leur utilisation pour catalyser la métathèse des huiles végétales a été largement moins étudiée. On peut trouver néanmoins les références suivantes :

La demande internationale WO-A-96/04289 (R. Grubbs et al.) décrit l'éthénolyse de l'oléate de méthyle avec des complexes de type 1 (Figure 1 ci-dessous). En présence d'un excès d'éthylène (100 psi) la réaction conduit à un mélange de décènes (43 %), de décénoate de méthyle (46 %) et en plus à des produits d'homométathèse (5 %). La demande internationale WO-A-99/51344 (W. Herrmann et al.), le brevet US 6 635 768 (Herrmann et al.), et la demande de brevet US 2004/0095792A1 (Herrmann et al.) décrivent l'utilisation de complexes analogues de type 2 (Figure 1 ci-dessous) pour catalyser l'homométathèse de l'oléate de méthyle et la métathèse de l'oléate de méthyle avec l'octène-1. Ces derniers complexes peuvent être plus actifs mais montrent également une activité isomérisante de la double liaison, se qui limite leur sélectivité pour la production d'oléfines alpha.

Le document Warwel et al., Chemosphère 43, 39-48 (2001) décrit la réaction de métathèse par éthénolyse de l'oléate de méthyle pour produire le décène-1 et le méthyl ester de l'acide 9-décénoïque.

La demande internationale WO-A-02/076920 (Newman et al.) décrit la mise en oeuvre de complexes du ruthénium de type 3 (Figure 1 ci-dessous) en milieu homogène ou supporté sur polymères, par exemple de type polystyrène. La particularité de ces complexes par rapport aux précédents est qu'ils portent un ligand chélate. Il apparaît clairement que l'immobilisation du complexe sur un support solide diminue considérablement l'activité du système.

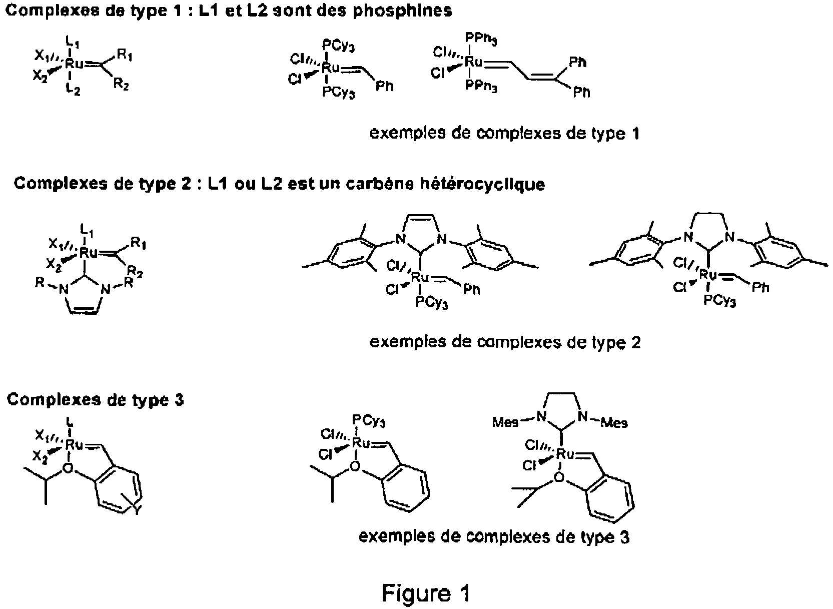

Ainsi, l'une des difficultés principales de ces systèmes à base de ruthénium est leur trop faible durée de vie. Leur mise en oeuvre en phase homogène donne les complexes les plus actifs, mais pose le problème de la séparation des produits de la réaction et de leur recyclage.

Une approche intéressante consiste à immobiliser le catalyseur dans une phase liquide (solvant) de laquelle on peut aisément séparer les produits, soit par distillation, soit par décantation si les produits sont peu miscibles avec le solvant.

Les liquides ioniques non-aqueux de formule générale O⁺A⁻ s'avèrent être des solvants particulièrement intéressants pour cette application. Ils présentent une très faible tension de vapeur (ne distillent pas) et des propriétés physico-chimiques modulables en fonction de l'anion et du cation qui les composent (voir par exemple H. Olivier-Bourbigou, L. Magna, J. Mol. Catal. A, Chem. 2002, vol. 182, p. 419).

L'immobilisation des catalyseurs à base de ruthénium dans les liquides ioniques a été décrite mais peu de littérature existe dans ce domaine. On peut citer par exemple le brevet EP-B-1 035 093. Cependant, les applications décrites ne concernent que des cas de métathèse par fermeture ou ouverture de cycle (RCM ou ROMP) et ne décrivent pas d'exemples de « cross »-métathèse.

L'éthénolyse des corps gras insaturés n'est pas décrite dans les liquides ioniques. Or il a été montré (B.R. Maughon et coll. Organometallics, 23, 2027, 2004) que :
- d'une part, la sélectivité de la réaction pour l'oléfine terminale (par exemple le décène-1) dépend du niveau de conversion du substrat (par exemple de l'oléate de méthyle) : plus la conversion est élevée, plus la sélectivité chute ;
- d'autre part, la perte d'activité du catalyseur pouvait être attribuée en partie à la présence, et à la concentration, de l'oléfine terminale dans le milieu.

Le développement d'un procédé d'éthénolyse des corps gras insaturés économiquement viable implique donc :
- de mettre au point un catalyseur stable, sélectif pour l'éthénolyse (éviter l'homométathèse) et peu isomérisant de la double liaison ;
- un procédé dans lequel l'oléfine alpha co-produite durant la réaction soit sélectivement extraite du milieu réactionnel, ceci afin d'éviter son effet inhibiteur sur le catalyseur ;
- un procédé dans lequel le catalyseur est recyclable et réutilisable.

L'invention a pour objet un procédé impliquant la métathèse de corps gras insaturés avec un excès d'éthylène, en présence d'un catalyseur comprenant au moins un composé du ruthénium et en présence d'au moins un liquide ionique non-aqueux.

L'invention a plus particulièrement pour objet un procédé de métathèse de corps gras choisis parmi les huiles de tournesol oléiques, les huiles de colza oléiques et les esters de monoalcools de ces huiles.

Dans ce nouveau procédé, le catalyseur (par exemple à base de complexe du ruthénium) est immobilisé et stabilisé dans le liquide ionique non-aqueux, dans lequel les oléfines produites sont très peu miscibles. Celles-ci sont donc extraites, pendant la réaction, et dès leur formation, dans une deuxième phase.

Dans ce nouveau procédé, les produits de la réaction peuvent être séparés aisément du liquide ionique contenant le catalyseur soit par distillation du fait de la non-volatilité du liquide ionique, soit par décantation du fait de la faible solubilité des oléfines formées dans le liquide ionique. Le catalyseur reste immobilisé et stabilisé dans le liquide ionique. Ce dernier contenant le catalyseur peut être recyclé et réutilisé.

Ce procédé est utilisé pour obtenir des compositions particulières de produits, qui seront séparés en plusieurs fractions distinctes ayant chacune un usage différent.

### La charge

Le procédé de métathèse de l'invention s'adresse à tout corps gras comprenant au moins un ester formé entre au moins un acide monocarboxylique par exemple d'au moins 12 atomes de carbone comportant au moins une insaturation éthylénique et au moins un composé aliphatique saturé hydroxylé (monoalcool ou polyol), le monoalcool étant par exemple un monoalcool de 1 à 8 atomes de carbone, et un polyol particulier étant le glycérol (cas des huiles végétales insaturées).

Plus particulièrement, le procédé de l'invention peut s'appliquer aux esters de l'acide oléique, acide gras dont la chaîne est porteuse d'une seule insaturation. Dans ce cas, la réaction d'éthénolyse conduit à la formation de seulement deux produits, le décène-1 et un ester de l'acide 9-décènoique. Cependant, il n'existe pas dans la nature de corps gras d'origine végétale ou animale dont les chaînes grasses seraient exclusivement constituées de chaînes oléiques. L'obtention d'un ester de l'acide oléique pur nécessite donc de recourir à une opération de séparation et de purification faisant le plus souvent appel à la distillation dans des conditions délicates et donc coûteuses.

Si l'une des oléfines est un ester d'acide gras di-insaturé, tel qu'un ester méthylique de l'acide linoléique et l'autre oléfine de l'éthylène, utilisé en un excès suffisant, la réaction de métathèse conduira à l'obtention d'une oléfine, l'heptène-1, d'une di-oléfine, le pentadiène-1,4 et d'un ester insaturé, le 9-décènoate de méthyle. La réaction peut s'écrire :

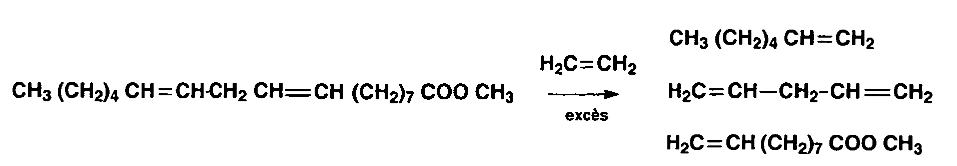

Dans cette réaction, on suppose que la réaction de métathèse par éthénolyse est complète, c'est-à-dire que toutes les insaturations de la chaîne linoléate ont réagi avec l'éthylène utilisé en excès. Cependant, une réaction de métathèse incomplète, c'est-à-dire impliquant une seule des deux insaturations de la chaîne linoléate, pourra conduire aux produits suivants :

**H₂C=CH-CH₂ CH =CH (CH₂)₇ COO CH₃**

**CH₃ (CH₂)₄ CH=CH CH₂ CH=CH₂**

On pourrait appliquer les mêmes réactions à toutes les chaînes insaturées d'acides gras connues, par exemple les chaînes d'acide tri-insaturées de type linolénique. Le nombre de produits potentiellement possible sera d'autant plus important que le nombre d'insaturations portées par la chaîne sera élevé.

La réaction de métathèse par éthénolyse peut également s'appliquer, non plus à un ester de monoalcool d'acide gras insaturé, mais au triglycéride correspondant. La réaction pourrait par exemple s'écrire :

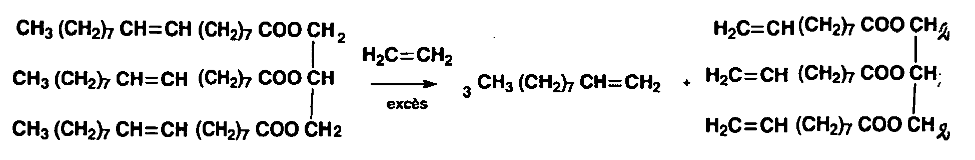

Si l'on applique cette réaction de métathèse par éthénolyse, non plus à une chaîne unique d'acide gras par exemple oléique ou linoléique comme précédemment, mais à un mélange de ces chaînes d'acides gras, comme c'est le cas dans la réalité lorsque l'on a affaire à des produits d'origine végétale ou animale, on obtiendra un mélange des produits issus de la métathèse par éthénolyse de chacune des chaînes grasses impliquées.

La nature des produits obtenus, ainsi que leur quantité dépendront donc de la composition en acides gras (nature et abondance) de la matière première grasse utilisée.

L'obtention de produits riches en décène-1 implique l'utilisation d'une matière première riche en esters de l'acide oléique.

L'huile végétale considérée (ou l'ester de monoalcool de cette huile) est choisie parmi l'huile de tournesol oléique ou l'huile de colza oléique (ou les esters de monoalcool de ces huiles). Ces huiles particulières et les esters de monoalcools dérivés de ces huiles sont caractérisés par leur composition en acides gras, notamment par la nature et la proportion de leurs acides gras insaturés. Dans ces huiles ou dans les esters de monoalcool de ces huiles, en général au moins 80 % des chaînes d'acides gras sont constituées de chaînes oléiques, la teneur en chaînes grasses linoléiques n'excède pas 12 % et la teneur en chaînes grasses linoléniques n'excède pas 0,3 %. Aucune autre chaîne oléfinique n'est présente dans ces huiles ou dans les esters de monoalcool de ces huiles à une teneur supérieure à 0,3 %, tandis que la teneur en chaînes saturées par exemple palmitique ou stéarique est comprise entre 5 % et 15 %.

Ces huiles peuvent être utilisées sous leur forme naturelle triglycéride ou sous la forme d'un mélange d'esters de monoalcool, comme par exemple le méthanol, l'éthanol, le propanol ou plus généralement tout monoalcool renfermant de 1 à 8 atomes de carbone.

Les réactions et les produits formés sont les suivants :
- dans le cas d'un ester d'un monoalcool représenté par exemple par du méthanol d'une huile de tournesol oléique ou de colza oléique : 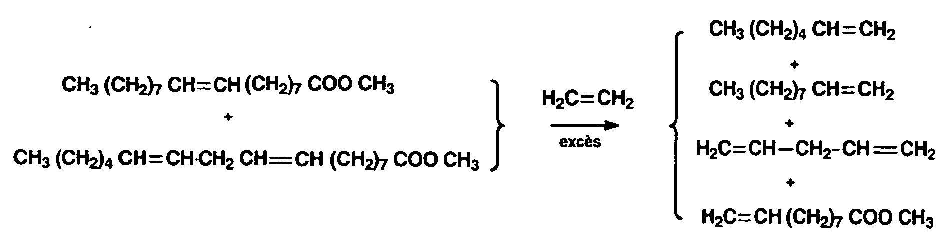
- dans le cas d'une huile de tournesol oléique ou d'une huile de colza oléique :

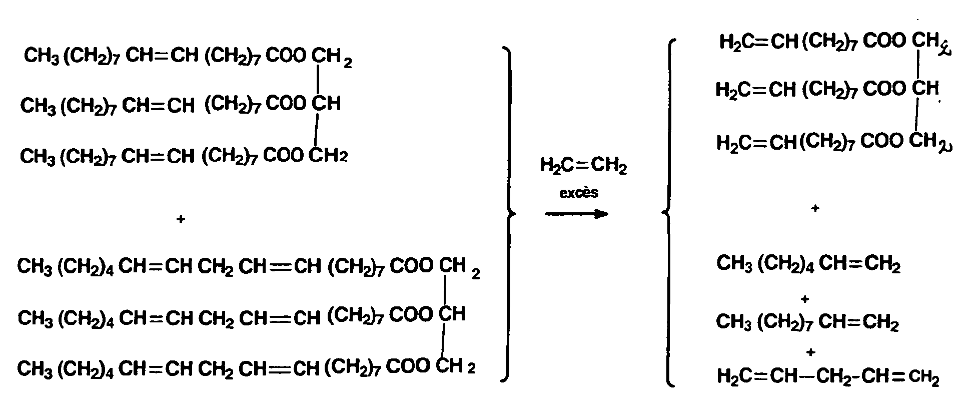

Dans tous les cas, les esters d'acides gras saturés présents dans l'huile de tournesol oléique ou l'huile de colza oléique ou dans les esters de monoalcools de ces huiles ne sont pas réactifs dans les réactions de métathèse et sont retrouvrés à la fin de l'opération. Ils ne sont donc pas représentés ici dans les équations.

Dans tous les cas, lorsque la réaction de métathèse est complète, on peut classer les produits obtenus en quatre familles :
- les monooléfines de type alpha-oléfines : décène-1 et heptène-1 ;
- une dioléfine, le pentadiène-1,4 ;
- les esters insaturés, qu'ils soient sous la forme 9-décènoate de monoalcool
ou sous la forme de triglycéride de l'acide 9-décènoique ;
- les esters saturés présents dans la matière première et qui ne sont pas concernés par la réaction de métathèse.

Si la réaction de métathèse n'est pas complète, s'ajouteront à ces produits des esters des acides oléique et linoléique résiduels sous la forme d'esters de monoalcool ou sous la forme de triglycérides.

Le procédé selon la présente invention peut comprendre une étape de séparation des oléfines par évaporation. En effet, on peut aisément séparer les monooléfines (décène-1 et heptène-1) et la dioléfine (pentadiène-1,4) du milieu réactionnel par évaporation, leurs températures d'ébullition respectives étant de 166,5°C, 94°C et 26°C, donc très inférieures à celle des esters présents et formés.

Dans le procédé selon la présente invention, on peut ensuite soumettre le mélange d'oléfines isolé précédemment à une distillation visant à séparer le 1,4-pentadiène, le 1-heptène et le 1-décène, ainsi que tout excès d'éthylène. L'éthylène en excès peut être réutilisé lors d'une nouvelle réaction de métathèse, tandis que chacune des autres mono- (ou di-)oléfines peut être valorisée et utilisée séparément.

Après évaporation de la fraction purement oléfinique (mono- et di-oléfines), le milieu réactionnel résiduel contient en conséquence un mélange d'esters, c'est-à-dire un ester de l'acide 9-décènoique sous la forme ester de monoalcool ou sous la forme triester du glycérol, selon la matière première utilisée (huile ou ester de monoalcool de cette huile), et aussi les esters des acides saturés présents dans la matière première, c'est-à-dire les esters des acides palmitique et stéarique sous la forme esters de monoalcool ou sous la forme triesters du glycérol, selon la matière première utilisée. Ces structures saturées n'étant pas concernées par la réaction de métathèse.

Cette fraction ester peut être définie par sa composition, qui découle de la composition de l'huile ou du mélange d'esters de monoalcool d'huile utilisé au départ.

A partir d'une huile telle que définie précédemment (huile de tournesol oléique ou l'huile de colza oléique) ou d'un mélange d'esters de monoalcool d'une de ces huiles, la composition en mono- et di-oléfines pourra être telle qu'indiquée plus loin.

Ainsi, par réaction de métathèse de l'huile de tournesol oléique ou de l'huile de colza oléique ou de leurs esters de monoalcool avec une oléfine particulière telle que l'éthylène, utilisé en excès, il est possible d'obtenir isolément :
- d'une part, une fraction oléfinique renfermant majoritairement (c'est-à-dire au moins 80 %) du décène-1, ainsi que de l'heptène-1 et du pentadiène-1,4 ;
- et d'autre part, une composition d'esters de monoalcool ou du glycérol riche en ester d'acide insaturé en C10.
   A partir d'une huile dont plus de 80 % des chaînes grasses sont constituées d'acide oléique ou d'un mélange d'esters de monoalcool d'une telle huile, une réaction de métathèse par éthénolyse conduira, si le rendement de cette réaction est au moins de 80 %,
- d'une part, à une fraction oléfinique majoritaire en décène-1 ;
- et d'autre part, après élimination par évaporation des mono- et di-oléfines, à un mélange d'esters dont plus de la moitié des chaînes sera constituée de chaînes insaturées en C10. Une telle composition ne correspond à aucun corps gras connu.

Un tel mélange d'esters est caractéristique en ce que sa concentration en chaînes insaturées en C10 est très élevée. Il est caractérisé également par la position de l'insaturation située entre l'atome de carbone en position 9 et celui en position 10 sur la chaîne carbonée. Cette position de l'insaturation est différente de celle observée dans les produits naturels.

On peut considérer par exemple un ester méthylique d'une huile de tournesol oléique dont la composition est la suivante :
- oléate de méthyle : environ 83 % en masse
- linoléate de méthyle : environ 10 % en masse
- palmitate de méthyle : environ 3 % en masse
- stéarate de méthyle : environ 4 % en masse.

Lorsque la réaction de métathèse est complète - c'est-à-dire lorsque chaque insaturation portée par les chaînes grasses aura réagi avec une mole d'éthylène selon le schéma réactionnel décrit précédemment -, elle conduira à un mélange ayant la composition suivante :
- décène-1 : environ 35,8 % en masse
- heptène-1 : environ 3,0 % en masse
- pentadiène-1,4 : environ 2,1 % en masse
- 9-décènoate de méthyle : environ 52,7 % en masse
- palmitate de méthyle : environ 2,75 % en masse
- stéarate de méthyle : environ 3,65 % en masse.

Une opération d'évaporation à une température inférieure à 180°C permettra d'isoler une coupe oléfinique ayant la composition suivante :
- décène-1 : environ 87,5 % en masse
- heptène-1 : environ 7,3 % en masse
- pentadiène-1,4 : environ 5,1 % en masse.

Ces composés pourront être séparés par distillation dans une étape ultérieure selon des méthodes connues.

La fraction ester non évaporée présentera la composition suivante :
- 9-décènoate de méthyle : environ 89,2 % en masse
- palmitate de méthyle : environ 4,6 % en masse
- stéarate de méthyle : environ 6,2 % en masse
   Cette fraction ester présente une composition tout à fait nouvelle. Aucun corps gras connu ne présente une telle composition.
   Si la matière première utilisée est une huile de tournesol oléique sous sa forme triglycéride, la fraction oléfinique sera comparable et les esters obtenus seront de type triglycérides au lieu d'être sous la forme d'ester méthylique. Dans ce cas, pour un même rendement de réaction, les compositions en acides gras seront très peu différentes.
   Dans le cas d'un mélange d'esters d'un monoalcool différent du méthanol, le rapport massique entre les fractions oléfinique et ester dépendra de la masse molaire du monoalcool considéré ; cependant le rapport molaire entre les produits obtenus restera identique, car il ne dépend que du rendement des réactions.
   Par hydrogénation totale ou partielle ultérieure des insaturations, un tel mélange conduira à un mélange d'esters dont la composition est comparable à celle des huiles de coprah ou de palmiste ou de leurs esters dérivés, dans le sens où la majorité des chaînes sont saturées et porteuses d'un nombre d'atome de carbone inférieur à 12. Les applications connues et possibles de ces huiles - dont la culture n'est pas pratiquée à grande échelle en Europe - ou de leurs dérivés pourront être envisagées pour les fractions esters issues de la métathèse des huiles de l'invention
ou de leurs esters de monoalcool. Ces applications concernent notamment la fabrication de savons par saponification, la fabrication d'acides gras par hydrolyse, etc.

Le décène-1 est un intermédiaire de synthèse très recherché. Il intervient notamment dans la fabrication de poly alpha oléfines qui sont des lubrifiants de synthèse ainsi que dans la préparation d'alcools et dans bien d'autres procédé de fabrication en chimie industrielle. Le décène-1 est généralement préparé par oligomérisation de l'éthylène, c'est-à-dire entièrement à partir d'une matière première d'origine fossile.

L'heptène-1 et le pentadiène-1,4 sont des intermédiaires de synthèse généralement issus du craquage de coupes pétrolières ou de charbon.

Par le procédé selon l'invention, ces composés sont donc accessibles, non plus uniquement à partir de matière première d'origine fossile, mais majoritairement à partir de matières premières renouvelables issues de la biomasse, comme l'huile de tournesol oléique ou l'huile de colza oléique. Par exemple une mole de décène-1 peut être obtenue à partir d'une chaîne d'acide gras et d'une demi-mole d'éthylène. Il faudrait 5 moles d'éthylène pour préparer 1 mole de décène-1 uniquement à partir de l'éthylène par oligomérisation de ce dernier.

### Le liquide ionique

Le solvant ionique non-aqueux est choisi dans le groupe formé par les sels liquides qui ont pour formule générale Q⁺ A- dans laquelle Q⁺ représente un ammonium quaternaire, d'un phosphonium quaternaire, un guanidium quaternaire ou un sulfonium quaternaire, et A- représente tout anion susceptible de former un sel liquide à basse température, c'est-à-dire en dessous de 90°C et avantageusement d'au plus 85°C, et de préférence en dessous de 50°C.

Les anions A⁻ sont de préférence choisis parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroéthyl)phosphate, hexafluoroantimonate, fluorosulfonate, alkylsulfonates (par exemple le méthylsulfonate), perfluoroalkylsulfonates (par exemple le trifluorométhylsulfonate), bis(perfluoroalkylsulfonyl)amidures (par exemple l'amidure de bis trifluorométhylsulfonyle de formule N(CF₃SO₂)₂⁻), le méthylure de tris-trifluorométhylsulfonyle de formule C(CF₃SO₂)₃⁻, le méthylure de bis-trifluorométhylsulfonyle de formule HC(CF₃SO₂)₃⁻, arènesulfonates, éventuellement substitués par des groupements halogènes ou halogénoalkyles, l'anion tétraphenylborate et les anions tétraphenylborates dont les noyaux aromatiques sont substitués, tétra-(trifluoroacétoxy)-borate, bis-(oxalato)-borate, dicyanamide, tricyanométhylure, ainsi que l'anion tétrachloroaluminate, ou les anions chlorozincates.

Les cations Q⁺ sont choisis dans le groupe des phosphoniums quaternaires, des ammoniums quaternaires, des guanidiniums quaternaires et des sulfoniums quaternaires.

Dans les formules ci-après, R¹, R², R³, R⁴, R⁵ et R⁶ représentent l'hydrogène (à l'exception du cation NH₄⁺ pour NR¹R²R³R⁴⁺), de préférence un seul substituant représentant l'hydrogène, ou des radicaux hydrocarbyles ayant de 1 à 30 atomes de carbone, par exemple des groupements alkyles, saturés ou non-saturés, cycloalkyles ou aromatiques, aryles ou aralkyles, éventuellement substitués, comprenant de 1 à 30 atomes de carbone.

R¹, R², R³, R⁴, R⁵ et R⁶ peuvent également représenter des radicaux hydrocarbyles portant une ou plusieurs fonctions choisies parmi les fonctions -CO₂R, -C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

Les cations Q⁺ ammonium et/ou phosphonium quaternaires répondent de préférence à l'une des formules générales NR¹R²R³R⁴⁺ et PR¹R²R³R⁴⁺, ou à l'une des formules générales R¹R²N=CR³R⁴⁺ et R¹R²P=CR³R⁴⁺ dans lesquelles R¹, R², R³ et R⁴, identiques ou différents, sont définis comme précédemment.

Les cations ammonium et/ou phosphonium quaternaires peuvent également être dérivés d'hétérocycles azotés et/ou phosphorés comportant 1, 2 ou 3 atomes d'azote et/ou de phosphore, de formules générales : 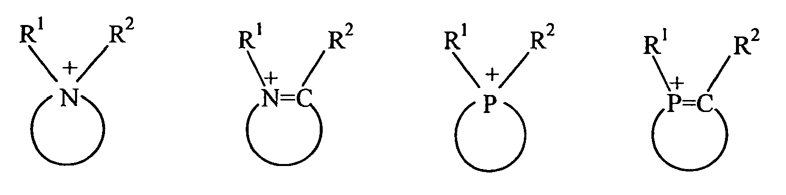 dans lesquelles les cycles sont constitués de 4 à 10 atomes, de préférence de 5 à 6 atomes, et R¹ et R², identiques ou différents, sont définis comme précédemment.

Le cation ammonium ou phosphonium quaternaire peut en outre répondre à l'une des formules générales :

R¹R²⁺N=CR³-R⁷-R³C=N+R¹R² et R¹R²+P=CR³-R⁷-R³C=P⁺R¹R²

dans lesquelles R¹, R² et R³, identiques ou différents, sont définis comme précédemment, et R⁷ représente un radical alkylène ou phénylène.

Parmi les groupements R¹, R², R³ et R⁴, on mentionnera les radicaux méthyle, éthyle, propyle, isopropyle, butyle primaire, butyle secondaire, butyle tertiaire, amyle, phényle ou benzyle ; R⁷ pourra être un groupement méthylène, éthylène, propylène ou phénylène.

De manière préférée, le cation ammonium et/ou phosphonium quaternaire Q⁺ est choisi dans le groupe formé par le *N*-butylpyridinium, le *N*-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le *N*-butyl-*N*-méthylpyrrolidinium, le *N*-butyl-*N*-méthylmorpholinium le triméthylphénylammonium, le tétrabutylphosphonium, le tributyl-tétradécylphosphonium.

Les cations sulfonium quaternaires et guanidinium quaternaires répondent de préférence à l'une des formules générales :

SR¹R²R³⁺ et C(NR¹R²)(NR³R⁴)(NR⁵R⁶)⁺

où R¹, R², R³, R⁴, R⁵ et R⁶, identiques ou différents, sont définis comme précédemment.

A titre d'exemples de sels utilisables selon l'invention, on peut citer le bis(trifluorométhylsulfonyl)amidure de butyl-3-méthyl-1-imidazolium, le bis(trifluorométhylsulfonyl)amidure de butyl-3-diméthyl-1,2-imidazolium, le bis(trifluorométhylsulfonyl)amidure de *N*-butyl-*N*-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le bis(trifluorométhylsulfonyl)amidure de (hydroxy-2-éthyl)-1-méthyl-3-imidazolium, le bis(trifluorométhylsulfonyl)amidure de (carboxy-2-éthyl)-1-méthyl-3-imidazolium, le bis(trifluorométhylsulfonyl)amidure de *N*-butyl-*N-*méthylmorpholinium. Ces sels peuvent être utilisés seuls ou en mélange.

### Les catalyseurs

Les catalyseurs utilisés dans le procédé de l'invention pour effectuer la réaction de métathèse des corps gras insaturés avec de l'éthylène en excès consistent en tout catalyseur comprenant au moins un composé du ruthénium.

Les catalyseurs au ruthénium sont choisis de préférence parmi les catalyseurs chargés ou non chargés de formule générale :

(X₁)ₐ(X₂)_{b}Ru(carbène C)(L₁)_{c}(L₂)_{d}

dans laquelle :
- a, b, c, d sont des nombres entiers avec a et b égaux à 0, 1 ou 2 ; c et d égaux à 0, 1, 2, 3 ou 4 ;
- X₁ et X₂, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénates, les amidures, le tosylate, l'hexafluorophosphate, le tétrafluoroborate, le bis-triflylamidure, le tétraphénylborate et dérivés.
   X₁ ou X₂ peuvent être liés à Y₁ ou Y₂ ou au (carbène C) de façon à former un ligand bidenté (ou chélate) sur le ruthénium ; et
- L₁ et L₂ identiques ou différents sont des ligands donneurs d'électrons tels que une phosphine, phosphite, phosphonite, phosphinite, arsine, stilbine, une oléfine ou un aromatique, un composé carbonylé, un ether, un alcool, une amine, une pyridine ou dérivé, une imine, un tioéthers, ou un carbène hétérocyclique qui répond par exemple à l'une des formules générales de la Figure 2, dans lesquelles R₁, R₂, R₃, R₄ et R₅ identiques ou différents, représentent chacun l'hydrogène, un groupe hydrocarboné, aliphatique, saturé ou insaturé, ou aromatique, comprenant de 1 à 12 atomes de carbone.

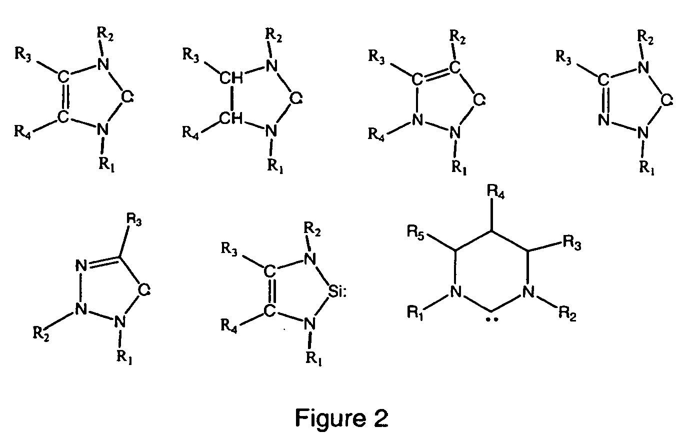

L₁ ou L₂ peuvent être liés au "carbène C " de façon à former un ligand bidenté ou chélate, comme indiqué dans la formule (Figure 3) dans laquelle Z représente un bi-radical hydrocarboné, aliphatique, cyclique ou non, saturé ou insaturé, ou aromatique, comprenant de 1 à 12 atomes de carbone ; Y est un hétéroélément tel que l'oxygène, l'azote, le soufre ou le phosphore.

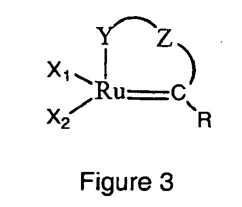

Le "carbène C" pourra être représenté par la formule générale : C(R₁)(R₂) pour laquelle R₁ et R₂ sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHR ou allénylidènes Ru=C=C=CR₁R₂ ou indénylidènes.

Un groupe fonctionnel permettant d'améliorer la rétention du complexe du ruthénium dans le liquide ionique peut être gréffé sur au moins l'un des ligands X₁, X₂, L₁, L₂, ou sur le carbène C. Ce groupe fonctionnel peut être chargé ou non chargé tel que de préférence un ester, un éther, un thiol, un acide, un alcool, une amine, un hétérocycles azoté, un sufonate, un carboxylate, un ammonium quaternaire, un guanidinium, un phosphonium quaternaire, un pyridinium, un imidazolium, un morpholinium ou un sulfonium.

Exemples de complexes fonctionnalisés : position possibles de la fonction

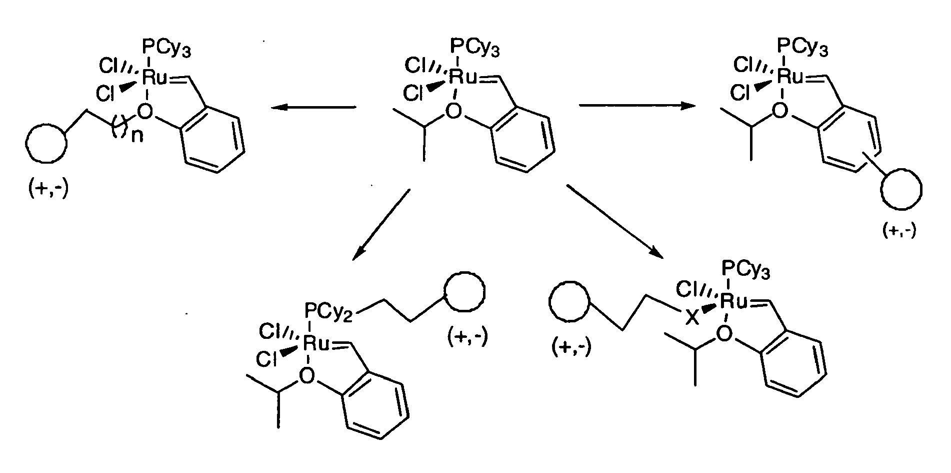

Parmi ces dérivés du ruthénium, on pourra citer les exemples suivants :

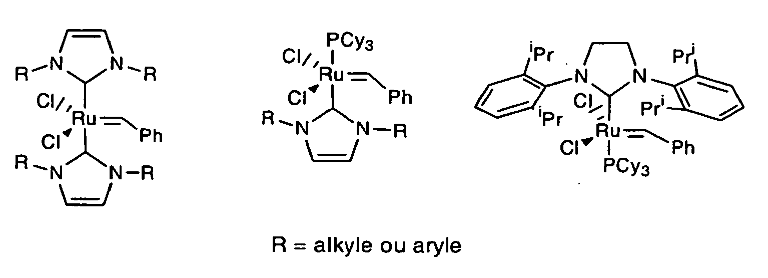

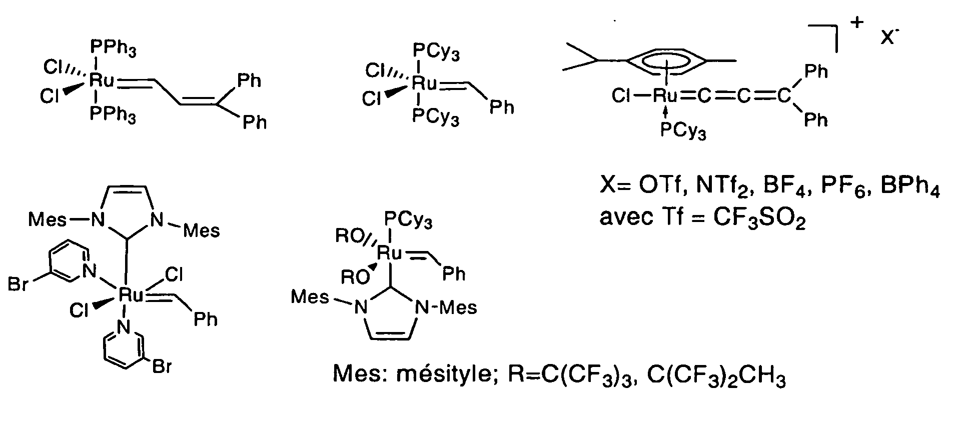

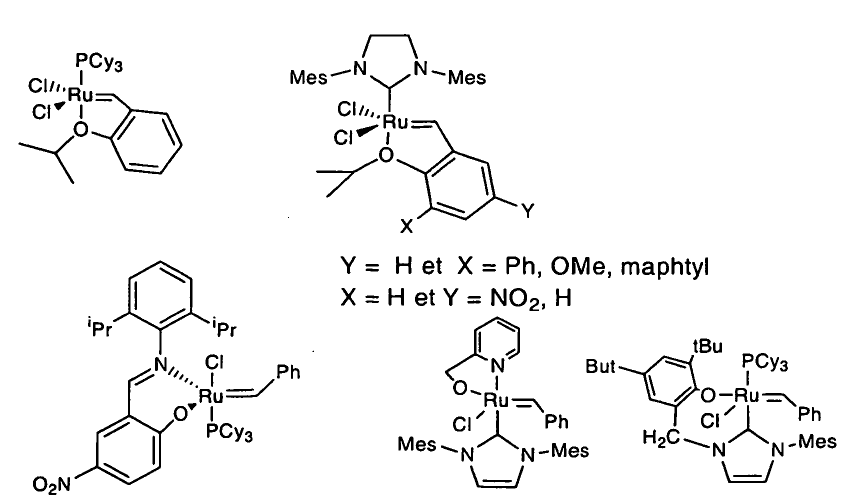

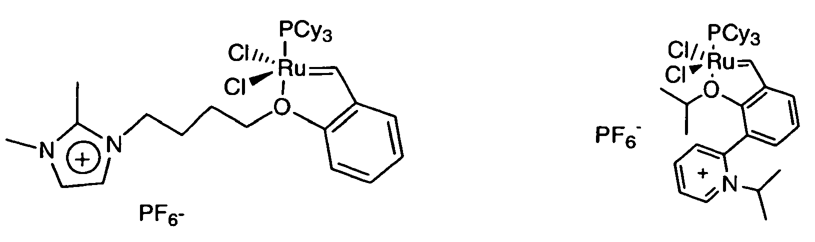

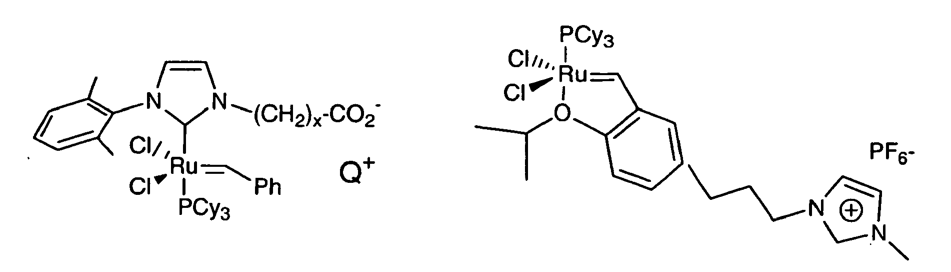

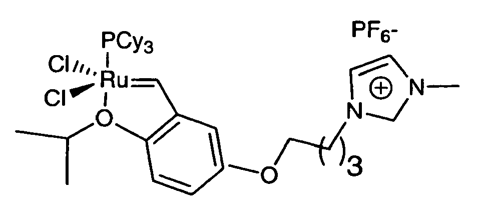

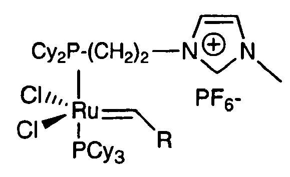

Dans ces formules Cy représente le radical cyclohexyle et iPr le radical isopropyle. Q+ représente un cation organique (par exemple ammonium, pyridinium, imidazolium ou phosphonium) ou inorganique (par exemple Na+, Ll+ ou K+).

### La mise en oeuvre

Dans le procédé de l'invention, le catalyseur est en général mis en jeu en une proportions comprise entre 0,001 % molaire et 10 % molaire par rapport au corps gras insaturé de départ. Préférentiellement, on introduira entre 0,01 % et 10 % molaire de catalyseur.

Selon le procédé de l'invention, la réaction de métathèse du corps gras de départ (par exemple l'huile de tournesol oléique ou de l'huile de colza oléique ou leurs esters de monoalcool) avec de l'éthylène utilisé en excès peut être conduite en l'absence ou en présence d'un co-solvant organique. Dans le cas où un solvant ou un mélange de solvants est utilisé, son rôle peut être d'améliorer la solubilisation des réactifs et du catalyseur dans le liquide ionique. Il peut aussi servir à optimiser l'extraction des produits dans une deuxième phase.

Parmi les solvants envisageables pour l'invention, on peut citer par exemple les chloroalcanes, tels que le dichlorométhane, le chloroforme ou les dichloro- ou trichloro-éthane, les solvants aromatiques tels que le toluène, les xylènes, ou le chlorobenzène, ou aliphatiques tel que l'heptane ou le cyclohexane.

Les réactions de métathèse de l'huile de tournesol oléique ou de l'huile de colza oléique ou de leurs esters de monoalcool avec de l'éthylène utilisé en excès peuvent être conduites en système fermé (batch), en système semi-ouvert ou en continu avec un ou plusieurs étages de réaction. On peut également envisager de réaliser la réaction en utilisant une distillation réactive.

Une vigoureuse agitation doit assurer un bon contact entre les réactifs (gazeux et liquide) et le mélange catalytique. La température de réaction peut être comprise entre 0°C et +150°C, de préférence entre 20°C et 120°C.

On peut opérer au-dessus ou en dessous de la température de fusion du milieu, l'état solide dispersé n'étant pas une limitation au bon déroulement de la réaction.

La pression peut être comprise par exemple entre la pression atmosphérique et 50 MPa.

L'éthylène peut être utilisé pur ou en mélange ou dilué avec une paraffine (inerte).

Les produits de la réaction peuvent être séparés par décantation.

Il est aussi possible de séparer les produits par distillation compte tenu de la non-volatilité du liquide ionique et de sa bonne stabilité thermique.

Les exemples suivants illustrent l'invention.

### Exemple 1 : Métathèse par éthénolyse de l'oléate de méthyle catalysée par un complexe de type 3 (Figure 1) dans un liquide ionique

Dans un réacteur autoclave équipé d'un système d'agitation et d'un capteur de pression, on introduit sous atmosphère inerte d'argon 1 mL de liquide ionique butyl-3-diméthyl-1,2-imidazolium bis-triflylamidure de formule : [BMMI]⁺ [N(CF₃SO₂)₂]⁻ pré-séché sous vide une nuit à 80°C, 148 mg d'oléate de méthyle (origine Fluka, de pureté supérieure à 98 %), et 15 mg de complexe de formule :

Cl₂Ru(=CH-*o*-O-iPrC₆H₄)PCy₃

(synthétisé par réaction du complexe de Grubbs 1^{ère} génération de formule: Cl₂Ru(=CHC₆H₅)(PCy₃)₂ avec le 1-isopropoxy-2-vinylbenzène en présence de CuCl), soit 5 % molaire de catalyseur par rapport à l'oléate de méthyle. L'autoclave est ensuite tiré sous vide puis pressurisé jusqu'à obtention d'une pression de 10 bar (1 MPa) d'éthylène (origine Alphagaz, qualité N25). La température est maintenue constante à 20°C.

Le milieu est agité à température ambiante pendant 2 heures, puis l'excès d'éthylène est lentement chassé par retour à la pression atmosphérique, à une température n'excédant pas 20°C et l'autoclave est remis sous atmosphère d'argon. Les produits sont séparés du liquide ionique par addition de 2 à 3 mL d'heptane distillé sur CaH₂ et dégazé. Un aliquote (100 *µ*L) de la solution extraite est passé sur une courte colonne de silice (2 cm) éluée à l'éther diéthylique. Il est analysé par chromatographie en phase gaz (colonne ZB-1, 100 % diméthylpolysiloxane, 30 mètres, gaz vecteur hélium 2 mL/min, programmation de température : 60°C puis 5°C/min jusqu'à 220°C) couplée à un spectromètre de masse.

La conversion de l'oléate de méthyle est de 95 %. Elle est calculée en utilisant le décane comme étalon interne. Les produits de la réaction sont composés de décène-1 et de décénoate de méthyle.

On ne détecte pas la présence d'isomères du décène-1. Les produits d'homométathèse sont présents à l'état de traces, non quantifiables.

### Exemple 2 : Recyclage du liquide ionique contenant le catalyseur

Après le premier cycle réalisé selon l'Exemple 1, l'autoclave contenant le liquide ionique et le catalyseur est tiré sous vide afin d'éliminer les traces d'heptane. Sous atmosphère d'argon, on rajoute 148 mg d'oléate de méthyle, puis on pressurise le réacteur jusqu'à obtention d'une pression de 10 bar (1 MPa) d'éthylène. On maintient la température à 20°C.

On réalise alors la même procédure que celle décrite dans l'Exemple 1 pour analyser les produits formés.

On réalise ainsi 3 cycles successifs sans rajouter de catalyseur ni de liquide ionique.

On détermine à chaque cycle la conversion de l'oléate de méthyle et la composition des produits formés (Tableau 1 ci-dessous).

**Tableau 1**

| | Conversion oléate de méthyle (% poids) | Produits formés |
|---|---|---|
| 1^{er} cycle (Exemple 1) | 95 | Décène-1 + décénoate de méthyle |
| 2^{ème} cycle | 95 | Décène-1 + décénoate de méthyle |
| 3^{ème} cycle | 85 | Décène-1 + décénoate de méthyle |

### Exemple 3 : Métathèse par éthénolyse de l'oléate de méthyle catalysée par un complexe de type 3 (Figure 1) dans un liquide ionique

On chauffe un réacteur autoclave de 50 mL à 50°C sous vide dynamique (0,2 hPa) pendant 4h. Après retour à température ambiante, on charge le réacteur avec 10 bar (1 MPa) d'éthylène.
- Après un dégazage effectué en début de test, on introduit 5 mL (7 g) de liquide ionique butyl-1-méthyl-1-pyrrolidinium bis(trifluorométhanesulfonyl)amide de formule [BMPyrr][NTf₂], ainsi qu'une solution de 1 g (3,37 mmol) d'oléate de méthyle et 80 mg d'octadécane (étalon interne pour la chromatographie) dans 10 mL d'heptane. On ajoute alors une solution de 49 mg (0,082 mmol) du complexe de formule :

   Cl₂Ru(=CH-*o*-O-iPrC₆H₄)PCy₃

   (synthétisé par réaction du complexe de Grubbs 1^{ère} génération de formule : Cl₂Ru(=CHC₆H₅)(PCy₃)₂ avec le 1-isopropoxy-2-vinylbenzène en présence de CuCl) dans 3 mL de toluène, ce qui correspond à 2,4 % molaire de catalyseur par rapport à l'oléate de méthyle. Le réacteur est placé sous une pression de 10 bar (1 MPa) d'éthylène pendant 2h 15 et la température est maintenue constante à 25°C. Le milieu réactionnel est agité au moyen d'un barreau magnétique (750 tr/min).

A l'issue de la réaction, le réacteur est dégazé et son contenu est intégralement prélevé dans une seringue en verre. La phase liquide ionique, plus dense, est immédiatement réinjectée dans le réacteur (sous pression atmosphérique d'éthylène) et la phase organique, après filtration sur une colonne de silice éluée au toluène est analysée par chromatographie en phase gaz.

La conversion de l'oléate de méthyle est de 95 %. Les produits de la réaction sont composés de décène-1 (rendement 79 %) et de décénoate de méthyle (rendement 72 %).

Des traces de produits d'isomérisation (< 1 %) sont détectées. On n'observe pas de produits de self-métathèse.

### Exemple 4 : Recyclage du liquide ionique contenant le catalyseur

A la phase liquide ionique issue du premier cycle de l'Exemple 3 on ajoute une solution de 1 g d'oléate de méthyle et 80 mg d'octadécane dans 10 mL d'heptane. Le réacteur est à nouveau placé sous une pression de 10 bar (1 MPa) d'éthylène pendant 2h 15 et la température est maintenue constante à 25°C.

A l'issue de la réaction, le contenu du réacteur est traité comme dans l'Exemple 3.

A l'issue du 2^{ème} cycle, la conversion de l'oléate de méthyle est de 61 %. Les produits de la réaction sont composés de décène-1 (rendement 57 %) et de décénoate de méthyle (rendement 58 %).

Des traces de produits d'isomérisation (< 1 %) sont détectées. On n'observe pas de produits de self-métathèse.

## Revendications

1. Procédé pour produire à la fois une fraction oléfinique et une composition d'esters **caractérisé en ce qu'**il comprend la réaction de métathèse, d'au moins un corps gras insaturé comprenant au moins un ester formé entre au moins un acide monocarboxylique comportant au moins une insaturation éthylénique et au moins un monoalcool aliphatique ou au moins un polyol aliphatique, avec de l'éthylène en excès, en présence d'un composé au ruthénium utilisé comme catalyseur et en présence d'au moins un liquide ionique non-aqueux choisi dans le groupe formé par les sels liquides en dessous de 90°C de formule générale Q⁺ A⁻ dans laquelle Q⁺ représente un phosphonium quaternaire, un ammonium quaternaire, un guanidinium quaternaire ou un sulfonium quaternaire et A' représente tout anion formant le sel liquide.

2. Procédé selon la revendication 1 **caractérisé en ce que** les cations Q⁺ ammonium ou phosphonium quaternaires, répondent à l'une des formules générales ;
NR¹R²R³R⁴⁺ et PR¹R²R³R⁴⁺,
ou à l'une des formules générales
R¹R²N=CR³R⁴⁺ et R¹R²P=CR³R⁴⁺
dans lesquelles R¹, R², R³ et R⁴, identiques ou différents, représentent l'hydrogène (à l'exception du cation NH₄⁺ pour NR¹R²R³R⁴⁺), des radicaux hydrocarbyles ayant de 1 à 30 atomes de carbone ou des radicaux hydrocarbyles portant une ou plusieurs fonctions choisies parmi les fonctions -CO₂R, -C(O)R, - OR, -C(O)NRR', -C(O)N(R)NR'R", -NRR', -SR, -S(O)R, -S(O)₂R, -SO₃R, -CN, - N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

3. Procédé selon la revendication 2 **caractérisé en ce que** les cations ammonium et/ou phosphonium quaternaires sont dérivés d'hétérocycles azotés et/ou phosphorés comportant 1, 2 ou 3 atomes d'azote et/ou de phosphore, de formules générales : 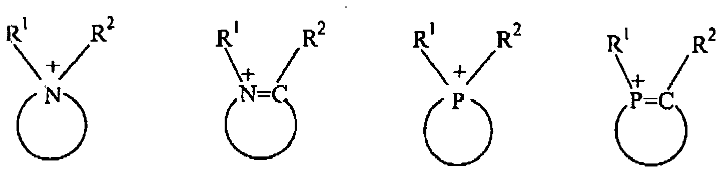 dans lesquelles les cycles sont constitués de 4 à 10 atomes, et R¹ et R², identiques ou différents, sont définis comme précédemment.

4. Procédé selon la revendication 1 **caractérisé en ce que** le cation ammonium ou phosphonium quaternaire répond à l'une des formules générales :
R¹R²⁺N=CR³R⁷-R³C=N⁺R¹R² et R¹R²⁺P=CR³-R⁷-R³C=P⁺R¹R²
dans lesquelles R¹, R² et R³, identiques ou différents, sont définis comme précédemment, et R⁷ représente un radical alkylène ou phenylène.

5. Procédé selon l'une des revendications 1 à 4 **caractérisé en ce que** le cation ammonium et/ou phosphonium quaternaire Q⁺ est choisi dans le groupe formé par le *N*-butylpyridinium, le *N*-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imïdazolium, le cation (hydroxy-2-éthyl)-1-méthyl-3-imidazolium, le cation (carboxy-2-éthyl)-1-méthyl-3-imidazolium, le diéthylpyrazolium, le *N*-butyl-*N*-méthylpyrrolidinium, le *N*-butyl-*N*-méthylmorpholinium le triméthylphénylammonium, le tétrabukylphosphonium et le tributyl-tétradécylphosphonium.

6. Procédé selon la revendication 1 **caractérisé en ce que** les cations sulfonium quaternaires et guanidinium quaternaires répondent à l'une des formules générales :
SR¹R²R³⁺ ou C(NR¹R²)(NR³R⁴)(NR⁵R⁶)⁺
où R1, R2, R3, R4, R5 et R6, identiques ou différents, sont définis comme R1, R2, R3 et R4 précédemment.

7. Procédé selon l'une des revendications 1 à 6 **caractérisé en ce que** les anions A-sont choisis parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroéthyl)phosphate, hexafluoroantimonate, fluorosulfonate, alkylsulfonates, perfluoroalkylsulfonates, bis(perfluoroalkylsulfonyl)amidures, le méthylure de tris-trifluorométhylsulfonyle de formule C(CF3SO2)3-, le méthylure de bis-trifluorométhylsulfonyle de formule HC(CF3SO2)3-, arènesuifonates, arènesulfonates substitués par des groupements halogènes ou halogénoalkyles, l'anion tétraphenylborate et les anions tétraphenylborates dont les noyaux aromatiques sont substitués, tétra-(trifiuoroacétoxy)-borate, bis-(oxalato)-borate, dicyanamide, tricyanométhylure, ainsi que l'anion tétrachloroaluminate, ou les anions chlorozincates.

8. Procédé selon l'une des revendications 1 à 6 **caractérisé en ce que** le liquide ionique est choisi parmi le bis(trifluorométhylsulfonyl)amidure de butyl-3-méthyl-1-imidazolium, le bis(trifluorométhylsulfonyl)amidure de butyl-3-diméthyl-1,2-imidazolium, le bis(trifluorométhylsulfonyl)amidure de *N*-butyl-*N-*méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le bis(trifluorométhylsulfonyl)amidure de (hydroxy-2-éthyl)-lwméthyl-3-imidazolium, le bis(trifluorométhylsulfonyl)amidure de (carboxy-2-éthyl)-1-méthyl-3-imidazolium et le bis(trifluorométhylsulfonyl)amidure de N-butyl-N-méthylmorpholinium.

9. Procédé selon l'une des revendications 1 à 8 **caractérisé en ce que** l'on soumet à la réaction de métathèse un corps gras insaturé comprenant au moins un ester formé entre un au moins un acide monocarboxylique comportant au moins une insaturation éthylénique et renfermant au moins 12 atomes de carbone et au moins un monoalcool aliphatique comportant de 1 à 8 atomes de carbone ou le glycérol.

10. Procédé selon la revendication 9 **caractérisé en ce que** l'on soumet à la réaction de métathèse un corps gras insaturé choisi parmi les huiles de tournesol oléiques et les huiles de colza oléiques, pour produire à la fois une fraction oléfinique et une composition d'esters de glycérol dont au moins une partie des chaînes est constituée de chaînes insaturées en C10.

11. Procédé selon la revendication 9 **caractérisé en ce que** l'on soumet à la réaction de métathèse un corps gras insaturé choisi parmi les mélanges d'esters de monoalcool des huiles de tournesol oléiques et des huiles de colza oléiques, pour produire à la fois une fraction oléfinique et une composition d'esters de monoalcool dont au moins une partie des chaînes est constituée de chaînes insaturées en C10.

12. Procédé selon la revendication 10 ou 11 **caractérisé en ce que** la composition en acides gras de l'huile de tournesol oléique a la composition en acides gras comprend environ :
· acide oléique : environ 83 % en masse
· acide linoléique : environ 10 % en masse
· acide palmitique : environ 3 % en masse et
· acide stéarique : environ 4 %, en masse

13. Procédé selon l'une des revendications 1 à 12 **caractérisé en ce que** le catalyseur est choisi parmi les catalyseurs chargés ou non chargés de formule générale :
(X₁)ₐ(X₂)_{b}Ru(carbène C)(L₁)_{c}(L₂)_{d}
dans laquelle :
• a, b, c, d sont des nombres entiers avec a et b égaux à 0, 1 ou 2 ; c et d égaux à 0, 1, 2, 3 ou 4 ;
• X₁ et X₂, identiques ou différents, représentent chacun un ligand mono- ou multi-chélatant, chargé ou non ; X₁ ou X₂ pouvant être liés à Y₁ ou Y₂ ou au carbène C de façon à former un ligand bidenté sur le ruthénium ; et
• L1 et L2 identiques ou différents sont des ligands donneurs d'électrons.

14. Procédé selon l'une des revendications 1 à 13 **caractérisé en ce que** le catalyseur est mis en jeu en une proportion comprise entre 0,001 % molaire et 10 % molaire par rapport au corps gras insaturé de départ.

15. Procédé selon l'une des revendications 1 à 14 **caractérisé en ce qu'**il comprend en outre une étape dans laquelle on sépare :
· une fraction oléfinique contenant des dioléfines et des monooléfines ;
· et une composition d'esters de monoalcool ou de glycérol.

16. Procédé selon la revendication 15 **caractérisé en ce que** la fraction oléfinique obtenue comprend au moins 80 % de décène-1.

17. Procédé selon la revendication 16 **caractérisé en ce que** la fraction oléfinique obtenue renferme, outre au moins 80 % de décène-1, de l'heptène-1 et du pentadiène-1,4.

18. Procédé selon la revendication 15 **caractérisé en ce qu'**il comprend en outre une étape dans laquelle les monooléfines et les dioléfines sont séparées de ladite fraction oléfinique par distillation.

19. Procédé selon la revendication 15 **caractérisé en ce que** plus de la moitié des chaînes de la composition d'esters de monoalcool ou de glycérol obtenue par séparation est constituée de chaînes insaturées en C10.

20. Procédé selon la revendication 19 **caractérisé en ce que** ledit monoalcool comporte de 1 à 8 atomes de carbone.

21. Procédé selon la revendication 20 **caractérisé en ce que** ledit monoalcool est le méthanol et **en ce que** la composition d'esters de monoalcool ou de glycérol comprend environ :
· 9-décènoate de méthyle : 89,2 % en masse ;
· palmitate de méthyle : 4,6 % en masse ; et
· stéarate de méthyle : 6,2 % en masse.

22. Procédé selon l'une des revendications précédentes dans lequel la température est comprise entre 0°C et +150°C, et la pression comprise entre la pression atmosphérique et 50 MPa.

## Claims

1. A process for producing both an olefinic fraction and an ester composition, **characterized in that** it comprises bringing at least one unsaturated fat comprising at least one ester formed between at least one monocarboxylic acid comprising at least one ethylenically unsaturated bond and at least one aliphatic monoalcohol or at least one aliphatic polyol into contact, under metathesis conditions, with an excess of ethylene in the presence of a catalyst and in the presence of at least one non-aqueous ionic liquid selected from the group formed by liquid salts which have general formula Q⁺A⁻ in which Q⁺ represents a quaternary ammonium, a quaternary phosphonium, a quaternary guanidinium, a quaternary sulfonium and A⁻ represents any anion which can form a liquid salt below 90°C.

2. A process according to claim 1, **characterized in that** the cations Q⁺, quaternary ammonium or phosphonium, have one of the following formulae; NR¹R²R³R⁴⁺ and PR¹R²R³R⁴⁺ or one of general formulae R¹R²N=CR³R⁴⁺ and R¹R²P=C R³R⁴⁺ in which R¹, R², R³ and R⁴, which may be identical or different, represent hydrogen (with the exception of the cation NH₄⁺ for NR¹R²R³R⁴⁺), hydrocarbyl radicals containing 1 to 30 carbon atoms or hydrocarbyl radicals carrying one or more functions selected from -CO₂R, -C(O)R, -OR, -C(O)NRR', -C(O)N(R)NR'R", -NR'R", -SR, -S(O)R, -S(O)₂R, -SO₃R, -CN, -N(R)P(O)R'R', -PRR', -P(O)RR', - P(OR)(OR'), -P(O)(OR)(OR') in which R, R' and R", which may be identical or different, each represent hydrogen or hydrocarbyl radicals containing I to 30 carbon atoms.

3. A process according to claim 2, **characterized in that** the quaternary ammonium and/or phosphonium cations may also be derived from nitrogen-containing and/or phosphorus-containing heterocycles comprising 1, 2 or 3 nitrogen and/or phosphorus atoms, with general formulae: 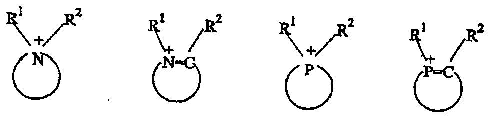 in which the cycles are constituted by 4 to 10 atoms and R¹ and R², which may be identical or different, are as defined above.

4. A process according to claim 1, **characterized in that** the quaternary ammonium or phosphonium cation has one of the following formulae:
R¹R²⁺N=CR³-R⁷-R³C=N⁺R¹R² and R¹R²⁺P=CR³-R⁷-R³C=P⁺R¹R²
in which R¹, R² and R³, which may be identical or different, are as defined above and R⁷ represents an alkylene or phenylene radical.

5. A process according to one of claims 1 to 4, **characterized in that** the quaternary ammonium and/or phosphonium cation Q⁺ is selected from the group formed by N-butylpyridinium, N-ethylpyridinium, pyridinium, 3-ethyl-1-methylimidazolium, 3-butyl-1-methylimidazolium, 3-hexyl-1-methylimidazoliuin, 3-butyl-1,2-dimethylimidazolium, the 1-(2-hydroxyethyl)-3-methylimidazolium cation, the 1-(2-carboxyethyl)-3-methylimidazolium cation, diethylpyrazolium, N-butyl-N-methylpyrrolidinium, N-butyl-N-methylmorpholinium, trimethylphenylammonium, tetrabutylphosphonium and tributyltetradecylphosphonium.

6. A process according to claim 1, **characterized in that** the quaternary sulphonium and quaternary guanidinium cations have one of the following general formulae:
S R¹R²R³⁺ or C(NR¹R²)(NR³R⁴)(NR⁵R⁶)⁺
in which R¹, R², R³, R⁴, R⁵ and R⁶, which may be identical or different, arc as defined above.

7. A process according to one of claims 1 to 6, **characterized in that** the anions A⁻ are selected from the following anions: halides, nitrate, sulphate, alkylsulphates, phosphate, alkylphosphates, acetate, halogenoacetatcs, tetrafluoroborate, tetrachloroborate, hexafluorophosphate, trifluoro-tris-(pentafluoroethyl)phosphate, hexafluoroantimonate, fluorosulphonate, alkylsulphonates, perfluoroalkylsulphonates, bis(perfluoroalkylphonyl)amides, tris-trifluoromethylsulphonyl methylide with formula C(CF₃SO₂)₃⁻, bis- trifluoromethylsulphonyl methylide with formula HC(CF₃SO₂)₃⁻, arenesulphonates, arenesulphonates substituted with halogens or halogenalkyl groups, the tetraphenylborate anion and tetraphenylborate anions the aromatic rings of which are substituted, tetra-(trifluoroacetoxy)-borate, bis-(oxalato)-borate, dicyanamide, tricyanomethylide, as well as the tetrachloroaluminate anion, or chlorozincate anions.

8. A process according to one of claims 1 to 6, **characterized in that** the ionic liquid is selected from 3-butyl-1-methylimidazolium bis(trifluoromethylsulphonyl)amide, 3-butyl-1,2-dimethylimidazolium bis(trifluoromethylsulphonyl)amide, N-butyl-N-methylpyrrolidinium bis(trifluoromethylsulphonyl)amide, 3-butyl-1-methylimidazolium tetrafluoroborate, 3-butyl-1,2-dimethylimidazolium tetrafluoroborate, 3-ethyl-1-methylimidazolium tetrafluoroborate, 3-butyl-1-methylimidazolium hexafluoroantimonate, 3-butyl-1-methylimidazolium trifluoroacetate, 3-ethyl-1-methylimidazolium triflate, 1-(2-hydroxyethyl)-3-methylimidazolium bis(trifluoromethylsulphonyl)amide, 1-(2-carboxyethyl)-3-methylimidazolium bis(tritluoromethylsulphonyl)amide and N-butyl-N-methylmorpholinium bis(tritluoromethylsulphonyl)amide.

9. A process according to one of claims 1 to 8, **characterized in that** an unsaturated fat comprising at least one ester formed between at least one monocarboxylic acid comprising at least one ethylenically unsaturated bond and containing at least 12 carbon atoms and at least one aliphatic monoalcohol containing 1 to 8 carbon atoms or glycerol undergoes the metathesis reaction.

10. A process according to claim 9, **characterized in that** an unsaturated fat selected from oleic sunflower oil and oleic rapeseed oil undergoes the metathesis reaction to produce both an olefinic fraction and a glycerol ester composition at least a portion of the chains of which is constituted by unsaturated C₁₀ chains.

11. A process according to claim 9, **characterized in that** an unsaturated fat selected from mixtures of monoalcohol esters of oleic sunflower oil and oleic rapeseed oil undergoes the metathesis reaction to produce both an olefinic fraction and a monoalcohol ester composition at least a portion of the chains of which is constituted by C₁₀ unsaturated chains.

12. A process according to claim 10 or claim 11, **characterized in that** the composition of oleic sunflower oil fatty acids in the fatty acid composition comprises approximately:
• oleic acid: about 83% by weight;
• linoleic acid: about 10% by weight;
• palmitic acid: about 3% by weight;
• stearic acid: about 4% by weight.

13. A process according to one of claims 1 to 12, **characterized in that** the catalyst is selected from charged or uncharged catalysts with general formula:
(X₁)ₐ(X₂)_{b}Ru(carbene C)(L₁)_{c}(L₂)_{d}
in which:
• a, b, c, d arc whole numbers in which a and b equal 0, 1 or 2; c and d equal 0, 1, 2, 3 or 4;
• X₁ and X₂, which may be identical or different, each represent a mono- or multi-chelating ligand, charged or uncharged; X₁ or X₂ may be bonded to Y₁ or Y₂ or to (carbene C) to form a bidentate ligand on the ruthenium; and
• L₁ and L₂, which may be identical or different, are donor electron ligands.

14. A process according to one of the claims 1 to 13 **characterized in that** the catalyst is introduced in a proportion comprised between 0.001% molar and 10% molar with respect to the unsaturated starting fat.

15. A process according to one of claims I to 14, **characterized in that** it further comprises a step in which the following are separated:
• an olefinic fraction containing diolefins and mono-olefins;
• and a composition of monoalcohol or glycerol esters.

16. An olefinic fraction according to claim 15, **characterized in that** it comprises at least 80% 1-decene.

17. A process according to claim 16, **characterized in that** the olefinic fraction comprises 1-heptene and 1,4-pentadiene in addition to at least 80% 1-decene.

18. A process according to claim 15, **characterised in that** it further comprises a step in which the mono-olefins and diolefins are separated from said olefinic fraction by distillation.

19. A process according to claim 15, **characterized in that** more than half of the chains of the composition of monoalcohol or glycerol esters obtained by separation is constituted by unsaturated C₁₀ chains.

20. A process according to claim 19, **characterized in that** said monoalcohol comprises 1 to 8 carbon atoms.

21. A process according to claim 20, **characterised in that** said monoalcohol is methanol and **in that** it comprises approximately:
• methyl 9-decenoate: about 89.2% by weight;
• methyl palmitate: about 4.6% by weight;
• methyl stearate: about 6.2% by weight.

22. A process according to one of the preceding claims, wherein the temperature is in the range 0°C to +150°C, and he pressure between atmospheric pressure and 50 MPa.

## Patentansprüche

1. Verfahren zur gleichzeitigen Herstellung einer Olefinfraktion und einer Esterzusammensetzung, **dadurch gekennzeichnet, dass** es die Metathesenreaktion mindestens eines ungesättigten Fettstoffs, der mindestens einen Ester umfasst, welcher aus mindestens einer mindestens einfach ethylenisch ungesättigten Monocarbonsäure und mindestens einem aliphatischen Monoalkohol oder mindestens einem aliphatischen Polyol gebildet ist, mit einem Überschuss an Ethylen in Gegenwart einer Rutheniumverbindung, die als Katalysator verwendet wird, und in Gegenwart mindestens einer nicht-wässrigen ionischen Flüssigkeit umfasst, wobei letztere aus den Salzen der allgemeinen Formel Q⁺A⁻ ausgewählt ist, welche bei weniger als 90 °C flüssig sind, wobei Q⁺ für ein quartäres Phosphonium, ein quartäres Ammonium, ein quartäres Guanidinium oder ein quartäres Sulfonium steht und A⁻ für ein beliebiges Anion steht, das ein flüssiges Salz bildet.

2. Verfahren nach Anspruch 1, **dadurch gekennzeichnet, dass** die quartären Ammonium- oder Phosphoniumkationen Q⁺ einer der folgenden allgemeinen Formeln:
NR¹R²R³R⁴⁺ und PR¹R²R³R⁴⁺,
oder einer der folgenden allgemeinen Formeln
R¹R²N=CR³R⁴⁺ und R¹R²P=CR³R⁴⁺
entsprechen, wobei R¹, R², R³ und R⁴, die identisch oder verschiedenartig sind, für Wasserstoff (mit Ausnahme des Kations NH₄⁺ für NR¹R²R³R⁴⁺), Kohlenwasserstoffreste mit 1 bis 30 Kohlenstoffatomen oder Kohlenwasserstoffreste stehen, die eine oder mehrere funktionelle Gruppen tragen, welche aus den funktionellen Gruppen -CO₂R, -C(O)R, - OR, -C(O)NRR', -C(O)N(R)NR'R", -NRR', -SR, -S(O)R, -S(O)₂R, -SO₃R, - CN, -N(R)P(O)R'R', -PRR', -P(O)RR', -P(OR)(OR'), -P(O)(OR)(OR') ausgewählt sind, wobei R, R' und R", die identisch oder verschiedenartig sind, jeweils für Wasserstoff oder Kohlenwasserstoffreste mit 1 bis 30 Kohlenstoffatomen stehen.

3. Verfahren nach Anspruch 2, **dadurch gekennzeichnet, dass** die quartären Ammonium- und/oder Phosphoniumkationen sich von stickstoffhaltigen und/oder phosphorhaltigen heterozyklischen Verbindungen ableiten, die 1, 2 oder 3 Stickstoff- und/oder Phosphoratome aufweisen, nach den folgenden allgemeinen Formeln: 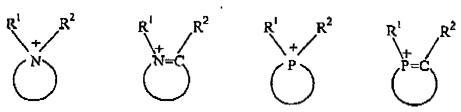 wobei die Ringe aus 4 bis 10 Atomen bestehen und R¹ und R², die identisch oder verschiedenartig sind, den obigen Begriffsbestimmungen entsprechen.

4. Verfahren nach Anspruch 1, **dadurch gekennzeichnet, dass** das quartäre Ammonium- oder Phosphoniumkation einer der folgenden allgemeinen Formeln:
R¹R²⁺N=CR³R⁷-R³C=N⁺R¹R² und R¹R²⁺P=CR³-R⁷-R³C=P⁺R¹R²
entspricht, wobei R¹, R² und R³, die identisch oder verschiedenartig sind, den obigen Begriffsbestimmungen entsprechen und R⁷ einen Alkylen - oder Phenylenrest darstellt.

5. Verfahren nach einem der Ansprüche 1 bis 4, **dadurch gekennzeichnet, dass** das quartäre Ammonium- und/oder Phosphoniumkation Q⁺ aus der Gruppe ausgewählt ist, die von *N*-Butylpyridinium, *N*-Ethylpyridinium, Pyridinium, 3-Ethyl-1-methylimidazolium, 3-Butyl-1-methylimidazolium, 3-Hexyl-1-methylimidazolium, 3-Butyl-1,2-dimethylimidazolium, dem 1-(2-Hydroxyethyl)-3-methylimidazoliumkation, dem 1-(2-carboxyethyl)-3-methylimidazoliumkation, Diethylpyrazolium, *N*-Butyl-*N-*methylpyrrolidinium, *N*-Butyl-*N*-methylmorpholinium, Trimethylphenylammonium, Tetrabutylphosphonium und Tributyltetradecylphosphonium gebildet wird.

6. Verfahren nach Anspruch 1, **dadurch gekennzeichnet, dass** die quartären Sulfonium- und quartären Guanidiniumkationen einer der folgenden allgemeinen Formeln:
SR¹R²R³⁺ oder C(NR¹R²)(NR³R⁴)(NR⁵R⁶)⁺
entsprechen, wobei R1, R2, R3, R4, R5 und R6, die identisch oder verschiedenartig sind, den obigen Begriffsbestimmungen für R1, R2, R3 und R4 entsprechen.

7. Verfahren nach einem der Ansprüche 1 bis 6, **dadurch gekennzeichnet, dass** die Anionen A⁻ aus den Anionen der Halogenide, Nitrat, Sulfat, der Alkylsulfate, Phosphat, der Alkylphosphate, Acetat, der Halogenacetate, Tetrafluorborat, Tetrachlorborat, Hexafluorphosphat, Trifluor-tris-(pentafluorethyl)phosphat, Hexafluorantimonat, Fluorsulfonat, der Alkylsulfonate, der Perfluoralkylsulfonate, der bis(Perfluoralkylsulfonyl)amide, tris-(Trifluormethylsulfonyl)methyl der Formel C(CF3S02)3-, bis-(Trifluormethylsulfonyl)methyl der Formel HC(CF3S02)3-, der Arensulfonate, der Arensulfonate, welche mit Halogen- oder Halogenalkylgruppen substituiert sind, dem Tetraphenylboratanion und den Tetraphenylboratanionen, deren aromatische Kerne substituiert sind, tetra-(Trifluoracetoxy)borat, bis-(Oxalato)-borat, Dicyanamid, Tricyanomethyl sowie Tetrachloraluminat, oder den Chlorzincatanionen ausgewählt sind.

8. Verfahren nach einem der Ansprüche 1 bis 6, **dadurch gekennzeichnet, dass** die ionische Flüssigkeit aus 3-butyl-1-methylimidazolium-bis(trifluormethylsulfonyl)amid, 3-Butyl-1,2-dimethyimidazolium-bis(trifluormethylsulfonyl)amid, *N*-Butyl-*N*-methylpyrrolidinium-bis(trifluormethylsulfonyl)amid, 3-Butyl-1-methyl-imidazolium-tetrafluorborat, 3-Butyl-1,2-dimethylimidazolium-tetrafluorborat, 3-Ethyl-1-methyl-imidazolium-tetrafluorborat, 3-Butyl-1-methyl-imidazolium-hexafluorantimonat, 3-Butyl-1-methyl-imidazolium-trifluoracetat, 3-Ethyl-1-methylimidazoliumtriflat, 1-(2-Hydroxyethyl)-3-methylimidazolium-bis(trifluormethylsulfonyl)amid, 1-(2-carboxyethyl)-3-methyl-3-imidazolium-bis(trifluormethylsulfonyl)amid und *N*-Butyl-*N-*methylmorpholinium-bis(trifluormethylsulfonyl)amid ausgewählt ist.

9. Verfahren nach einem der Anspruche 1 bis 8, **dadurch gekennzeichnet, dass** ein ungesättigter Fettstoff, der mindestens einen Ester umfasst, welcher aus mindestens einer mindestens einfach ethylenisch ungesättigten Carbonsäure mit mindestens 12 Kohlenstoffatomen und mindestens einem aliphatischen Monoalkohol mit 1 bis 8 Kohlenstoffatomen oder Glycerin gebildet ist, der Metathesenreaktion unterzogen wird.

10. Verfahren nach Anspruch 9, **dadurch gekennzeichnet, dass** ein ungesättigter Fettstoff, der aus den ölsäurereichen Sonnenblumenölen und den olsäurereichen Rapsölen ausgewählt ist, der Metathesenreaktion unterzogen wird, um gleichzeitig eine Olefinfraktion und eine Glycerinesterzusammensetzung herzustellen, wobei mindestens ein Teil der Ketten aus ungesättigten C10-Ketten besteht.

11. Verfahren nach Anspruch 9, **dadurch gekennzeichnet, dass** ein ungesättigter Fettstoff, der aus den Gemischen der Monoalkoholester ölsäurereicher Sonnenblumenöle und ölsäurereicher Rapsöle ausgewählt ist, der Metathesenreaktion unterzogen wird, um gleichzeitig eine Olefinfraktion und eine Monoalkoholesterzusammensetzung herzustellen, wobei mindestens ein Teil der Ketten aus ungesättigten C10-Ketten besteht.

12. Verfahren nach Anspruch 10 oder 11, **dadurch gekennzeichnet, dass** die Fettsäurezusammensetzung des ölsäurereichen Sonnenblumenöl ungefähr die Fettsäurezusammensetzung hat, welche Folgendes umfasst
| | |
|---|---|
| Ölsäure. | ungefähr 83 Massen-% |
| Linolsäure: | ungefähr 10 Massen-% |
| Palmitinsäure: | ungefähr 3 Massen-% und |
| Stearinsäure: | ungefähr 4 Massen-% |

13. Verfahren nach einem der Ansprüche 1 bis 12, **dadurch gekennzeichnet, dass** der Katalysator aus den geladenen oder ungeladenen Katalysatoren der folgenden allgemeinen Formel ausgewählt ist:
(X₁)ₐ(X₂)_{b}Ru(Carben C)(L₁)_{c}(L₂)_{d}
wobei
• a, b, c, d ganze Zahlen sind, wobei a und b gleich 0, 1 oder 2 sind; c und d gleich 0, 1, 2, 3 oder 4 sind;
• X₁ und X₂, die identisch oder verschiedenartig sind, jeweils einen einfach oder mehrfach chelatisierenden Liganden darstellen, der geladen ist oder nicht; wobei X₁ oder X₂ derart an Y₁ oder Y₂ oder an das Carben C gebunden sein können, dass ein zweizähniger Ligand am Ruthenium gebildet wird; und+
• L1 und L2, die identisch oder verschiedenartig sind, elektronenspendende Liganden sind.

14. Verfahren nach einem der Ansprüche 1 bis 13, **dadurch gekennzeichnet, dass** der Katalysator zu einem Anteil eingesetzt wird, der im Bereich von 0,001 Mol-% bis 10 Mol-% liegt, bezogen auf den ungesättigten Ausgangsfettstoff.

15. Verfahren nach einem der Ansprüche 1 bis 14, **dadurch gekennzeichnet, dass** es darüber hinaus einen Schritt umfasst, bei welchem
- eine Olefinfraktion, die Diolefine und Monoolefine enthält;
- und eine Monoalkohol- oder Glycerinesterzusammensetzung
voneinander getrennt werden.

16. Verfahren nach Anspruch 15, **dadurch gekennzeichnet, dass** die erhaltene Olefinfraktion mindestens 80 % an 1-Decen umfasst.

17. Verfahren nach Anspruch 16, **dadurch gekennzeichnet, dass** die erhaltene Olefinfraktion, neben mindestens 80 % an 1-Decen, 1-Hepten und 1,4-Pentadien umfasst.

18. Verfahren nach Anspruch 15, **dadurch gekennzeichnet, dass** es darüber hinaus einen Schritt umfasst, in welchem die Monoolefine und die Diolefine mittels Destillation von der Olefinfraktion abgetrennt werden.

19. Verfahren nach Anspruch 15, **dadurch gekennzeichnet, dass** mehr als die Hälfte der Ketten der Monoalkohol- oder Glycerinesterzusammensetzung, die bei der Abtrennung erhalten wird, aus ungesättigten C10-Ketten besteht.

20. Verfahren nach Anspruch 19, **dadurch gekennzeichnet, dass** der Monoalkohol 1 bis 8 Kohlenstoffatome aufweist.

21. Verfahren nach Anspruch 20, **dadurch gekennzeichnet, dass** es sich bei dem Monoalkohol um Methanol handelt, und dadurch, dass die Monoalkohol- oder Glycerinesterzusammensetzung ungefähr
• Methyl-9-decenoat 89,2 Massen-%;
• Methylpalmitat: 4,6 Massen-%; und
• Methylstearat: 6,2 Massen-%
umfasst.

22. Verfahren nach einem der vorhergehenden Ansprüche, wobei die Temperatur im Bereich von 0 °C bis +150 °C liegt und der Druck zwischen dem Atmosphärendruck und 50 MPa einschließlich liegt.
